# EUROPEAN PATENT SPECIFICATION

(11) **EP 2 405 886 B1**
(45) Date of publication and mention of the grant of the patent: **27.09.2017**
(21) Application number: 10751414.3
(22) Date of filing: 11.03.2010
(51) Int. Cl.: A61K 8/365, A61K 8/368, A61K 8/81, A61Q 19/00, A61Q 19/08

(54) **TOPICAL PERSONAL CARE AND PHARMACEUTICAL COMPOSITIONS AND USES THEREOF**
TOPISCHE KÖRPERPFLEGE- UND PHARMAZIEZUSAMMENSETZUNGEN SOWIE ANWENDUNGEN DAVON
COMPOSITIONS DE SOINS PERSONNELS ET PHARMACEUTIQUES TOPIQUES ET LEURS UTILISATIONS

(30) Priority: 11.03.2009 US 159284 P; 17.04.2009 US 170278 P
(43) Date of publication of application: 18.01.2012
(73) Proprietor: Isp Investments Inc., Wilmington, DE 19805 (US)
(72) Inventor: HANI, Fares, Somerset NJ 08873 (US); CHRIS, Barrett, Oakland NJ 07436 (US); TRACEY, Ross, Hewitt NJ 07421 (US); ANTHONY, Luschen, Wayne NJ 07470 (US)
(74) Representative: Harrison, Susan Joan
(86) International application number: PCT/US2010/026976
(87) International publication number: WO 2010/105052

(56) References cited:
- WO-A1-98/52536
- US-A- 73 614
- US-A- 5 139 770
- US-A- 5 736 128
- US-A1- 2004 234 491
- US-A1- 2008 113 037
- US-B1- 6 312 714
- US-B1- 6 312 714
- DATABASE GNPD [Online] MINTEL; 1 August 2008 (2008-08-01), "Anti-Ageing Elixir", XP002682902, Database accession no. 961639
- DATABASE GNPD [Online] MINTEL; 1 January 2009 (2009-01-01), "Advanced Contour Repositioning Serum", XP002682903, Database accession no. 1038188
- "CARBOPOL AQUA SF-1 POLYMER . SALICYLIC ACID SHAMPOO FORMULATION", INTERNET CITATION, December 2000 (2000-12), XP001240406, Retrieved from the Internet: URL:http://www.personalcare.noveon.com/pro ducts/carbopol/carbopolSF1.asp [retrieved on 2006-01-01]

## Description

### FIELD OF THE INVENTION

The present invention relates to topical compositions comprising at least one personal care acid or one pharmaceutical acid, which is an alpha beta or beta hydroxy acid and lightly- to moderately crosslinked poly(*N-*vinyl-2-pyrrolidone) ("PVP"). The lightly- to moderately crosslinked PVP has been found to provide unique thickening effects in acidic systems that are essentially stable (*e.g.,* do not phase separate and maintain rheological properties) even with prolonged storage.

Particularly, the invention relates to the compositions having 0,5% (% w/w) or more of the at least one personal care acid or pharmaceutical acid. These compositions ideally have an acidic pH, especially a pH less than 6, and more preferably a pH less than 4, and especially preferably less than 2. These formulations find application on the skin, hair, scalp, foot, or lip of an mammal, preferably man, as a smoothing composition, a moisturizing composition, a skin firming composition, a skin lightening composition, an age-spot composition, a shampoo, or a cream for use around the eyes or mouth.

Surprisingly, the topical compositions described herein deliver the personal care and/or pharmaceutical acid with reduced skin irritation, a significant breakthrough in this field where discomfort issues are well known.

### DESCRIPTION OF RELATED ART

Topical personal care and pharmaceutical compositions are products consumers around the globe have come to depend and rely on for the innumerable benefits they impart. Sold both by prescription and over-the-counter (non-prescriptive), they are applied to the exterior of the body to the skin, scalp, hair, feet, and lips. They may be cosmetic in effect, meaning they impart primarily aesthetically beneficial results (like minimizing fine lines and wrinkles), or they may relieve or cure clinical conditions (like acne vulgaris or warts), or fall somewhere between the cosmetic and medical indications. Across all these uses, many different product forms are employed, and vary from thickened "semi-solids" like foundations, concealers, lipsticks, and lip balms, to creamy emulsions, gels, ointments, and lotions, or may be lighter "bodied" compositions such as liquid soaps, washes, and rinses. In short, topical personal care and pharmaceutical compositions are ubiquitous in today's modem world.

It has been known for some time that acidic personal and pharmaceutical compositions elicit special responses when applied topically. In this broad concept, the term *low pH* means having a pH of 6 or less. More particularly, low pH compositions can cause an increase in epidermis exfoliation to alleviate skin conditions (e.g., hyperkeratosis, dry/flaky/itchy skin), enhance moisturization to help minimize the appearance of lines and wrinkles, increase dermal thickness, and increase dermal perfusion (vascular effects). A review of these actions as related to a particular type of acids, hydroxy acids and retinoids, is provided in Ramos-e-Silva, et al., "Hydroxy acids and retinoids in cosmetics," Clinics in Dermatog., 2001; 19:460-466. Also, an instructive review of alpha hydroxy acids, including the types, mechanisms of action, formulations, and treatment results, is provided by Van Scott, E.J., "Alpha-hydroxyacids in the treatment of signs of photoaging," Clinics in Dermat., 1996; 14: 217-226. This article recognizes pHs in the range from 0.6 to 4.0.

While low pH topical compositions can provide useful benefits to the consumer, they can pose real challenges to the formulation scientist, production staff, and even the consumer, It is well appreciated by one skilled in the art that low pH fluids can be difficult to thicken, or to maintain a stable viscosity and/or pH. Thickeners commonly used in low pH systems include xanthan gum and magnesium aluminum silicate combinations. At addition levels to create "thick" or "stiff" consistencies, these thickeners may cause pilling (localized formulary incompatibility that leads to coagulation) or impart an unpleasant, stringy texture to the end product.

Alternatively, acrylic acid polymers, and polyacrylamides may be used. Their manufacturers usually recommend dispersing them in water and then neutralizing to attain a desired viscosity target, which simply is not possible when the product inherently remains strongly acidic.

Other thickeners are known. For example, Carbopol^{®} Aqua SF-1, a lightly crosslinked acrylate copolymer is sold by The Lubrizol Corporation. Product information indicates it is effective at a pH of 3.5 and higher. Also sold by The Lubrizol Corporate is Carbopol^{®} Aqua CC Polymer, a polyacrylate-1 crosspolymer. The product white paper recommends neutralizing the polymer between a pH of 3.5 to 4.0, and, optionally, the pH can be adjusted (higher) by the addition of base. However, there still remains a need for a thickening agent that is effective at pHs of 6 or less, more preferably at very low pHs of 4 or less, and especially at extremely low pH of 2 or less.

Also known is U.S. patent 5,422,112, which discloses a thickener system including a combination of xanthan gum, magnesium aluminum silicate and polyacrylamide. The compositions are the to be particularly effective at low pH used especially for thickening alpha-hydroxy carboxylic acids and salts thereof. Typically, magnesium aluminum silicates have a recommended pH range of about 4.2 to 5.2, and typically are not the choice thickener for very low pH systems.

Similarly, U.S. patent 5,874,095 claims an enhanced skin penetration system comprising a nonionic polyacrylamide of high molecular weight, for improved topical delivery of drugs at low pH.

Further descriptions of acrylic acid thickeners are given in U.S. patents 2,883,351; 2,956,046; 3,035,004; and 3,436,378.

Poly(*N-*vinyl-2-pyrrolidone) and its salts and esters are described in U.S. patents 6,436,380; 6,197,281; 6,333,039; 6,685,952; and 7,108,860 as rheology modifiers or thickeners in personal care products.

U.S patent application 2003/0118620 teaches a thickening system for cosmetic composition of low pH, comprising a polysaccharide and taurate copolymer.

Polymeric thickeners for acidic surfactant compositions are described by U.S. patent 4,552,685, and by U.S. patent 4,529,773. However, these acidic-thickened solutions require high levels of surfactant in order to solubilize the copolymers and they have higher viscosities at pH 7 than when the pH is lowered into the acidic region.

As shown in this summary, there remains a strong demand and need for a thickening material for low pH, very low pH, and extremely low pH systems, particularly one that maintains stable viscosity, pH, and preferably viscosity and pH. Preferably, this thickener is easy to handle, readily dispersible, and provides smooth, thickened consistencies, without being stringy or creating pilling.

Interest in thickening acidic compositions stems, in part, from the growth of acid products that consumers are demanding and using. Although the use of alpha hydroxy acids as therapy for photoaged skin was known to medical doctors by 1989 (Van Scott, E.J., "Alpha hydroxy acids: procedures for use in clinical practice, Cutis, 1989; 43: 222-228), a non-prescriptive market demand did not exist until 1992, when Avon launched Anew *Perfecting Complex For Face* (Avon Products, Inc. website: www.avoncompany.com/brands/skincare.html). Indeed, the U.S. Food and Drug Administration (FDA) confirms that it was not until 1992 that they received the first four registrations for new consumer products containing glycolic acid as an active ingredient (Barrows, J.N., Memorandum to the Administrative File, "Guidance for Industry: Labeling for Topically Applied Cosmetic Products Containing Alpha Hydroxy Acids as Ingredients," Office of Cosmetics and Colors, CFSAN, FDA, September 12, 2002.) Market demand for these low pH, topically applied products grew such that by 1997 forty-two such product registrations were received by the FDA.

With the growth of this new market segment, consumers began to experience potentially harmful side effects like stinging, redness, and burning. Between 1992 and 2004 the FDA received 114 side-effect complaints (U.S. Food and Drug Administration, *Guidance: Labeling for cosmetics containing alpha hydroxy acids,* http://www.cfsan/fda/gov/guidance.html, January 10, 2005). Hence, there remains a real need for products and methods for reducing the irritation of these products while maintaining their efficacy in treating various skin and hair conditions.

As it will be explained later, the present invention is also related to lightly- to moderately-crosslinked poly(*N-*vinyl-2-pyrrolidone). This polymer was first introduced in U.S. patent 5,073,614. In that patent it is taught to be the precipitation polymerization product of *N*-vinyl-2-pyrrolidone monomer in an organic solvent, such as an aliphatic hydrocarbon solvent (preferably cyclohexane or heptane) or an aromatic hydrocarbon (such as toluene) in the presence of about 0.2% to 1% by weight of a crosslinking agent. The fine, white powders thus produced have an aqueous gel volume of about 15 mL to 150 mL of polymer, and a Brookfield viscosity in 5% aqueous solution of at least about 10,000 cP.

This lightly- to moderately-crosslinked poly(*N-*vinyl-2-pyrrolidone) polymer also was the subject of U.S. patent 5,139,770, filed December 17, 1990 and issued August 18, 1992. In this patent examples are provided for a cream rise (pH of 4), a hair conditioner (pH of 4), and a blow dry styling lotion (pH of 6), which have been pH-adjusted by the addition of citric acid or phosphoric acid. Although not specified, one skilled in the art recognizes that the acid addition level in these formulations is small, much less than 0.5% (% w/w). As such, formulation scientists regard these acids at these levels not as *functional* acids (*e.g.,* for the *treatment* of skin or hair conditions), but, instead as *pH adjustors,* necessary to protonate the quaternary polymer(s) to make them more substantive to hair.

U.S. patent 5,716,634 teaches a lightly-crosslinked *N*-vinyl lactam polymer in form of stable, clear, flowable, homogenized hydrogel, may be used as a carrier for cosmetic/pharma active for hair or skin use. A controlled release drug-delivery composition comprising a lightly-crosslinked poly(*N*-vinyl-2-pyrrolidone) polymer is the subject of U.S. patent 5,252,611. Also, the production of lightly-crosslinked poly(*N*-vinyl-2-pyrrolidone) polymer in an oil-in-water or water-in-oil emulsion is taught in U.S. patent 6,177,068.

A summary of some properties of light- to moderately-crosslinked poly(*N*-vinyl-2-pyrrolidone) is given in Shih, J.S., "Characteristics of lightly crosslinked poly(N-vinylpymolidone)," Polymer Materials: Science & Engineering Preprint, 72, 374, 1995.

Still more information on this lightly crosslinked poly(*N*-vinyl-2-pyrrolidone) polymer is given in the following U.S. patents: 5,162,417; 5,312,619; 5,622,168; 5,564,385; and 6,582,711.

Hence, a first objective of the present invention is to provide a wide range of easy-to-use, topical compositions having at least one personal care or pharmaceutical acid that are effectively thickened. The invention also seeks the use of these compositions to deliver the personal care/pharmaceutical acid(s), to reduce the perceived irritation and sting discomfort so these compositions find greater efficacy and consumer appeal.

### SUMMARY OF THE INVENTION

Surprisingly, it has been discovered that lightly- to moderately-crosslinked PVP effectively and quite elegantly thickens topical compositions having a personal care or pharmaceutical acid, even at a low pH of 6 or less, or very low pHs of 4 or less, or even extremely low pHs of 2 or less.

Additionally and even more surprising, it has been discovered that the use of these topical compositions thickened with lightly- to moderately-crosslinked PVP reduce irritation and sting discomfort compared to formulas without the lightly- to moderately-crosslinked PVP.

Hence, a first object of the present invention is to provide a thickener system particularly suited for use with acidic topical compositions, wherein the thickening agent comprises lightly- to moderately-crosslinked PVP. The topical compositions are those compositions for use on the exterior (*i.e.,* skin, hair, feet, and/or lips) of an mammal, such as man, horses, cats, and dogs. These thickened compositions serve both prescriptive and non-prescriptive markets, such as pharmaceutical and personal care compositions for skin care, hair care, foot care, scalp care, and sun care.

In these topical compositions the amount of lightly- to moderately-crosslinked PVP represents from 1% to 6% by weight. At these addition levels the low-shear ("Brookfield") viscosity typically is about 7000 cP or more, and more typically is about 10,000 cP or more.

A second objective of the present invention is the use of these thickened, acidic compositions to deliver the personal care and/or pharmaceutical acid to the exterior of a mammal, and to use this method to reduce irritation and sting compared to compositions not having the lightly- to moderately-crosslinked PVP.

### BRIEF DESCRIPTION OF THE DRAWINGS

Figure 1 is a graph of viscosity as a function of time for an acne gel produced in accordance with Example 8.
Figure 2 is a graph of pH as a function of time for an acne gel produced in accordance with Example 8.

### DESCRIPTION OF PREFERRED EMBODIMENTS OF THE INVENTION

The present invention relates to compositions comprising at least one personal care or pharmaceutical acid, which is an alpha beta or beta hydroxy acid and lightly- to moderately-crosslinked poly(*N-*vinyl-2-pyrrolidone) ("lightly-to moderately-crosslinked PVP") to thicken the composition as defined in claim 1. Surprisingly, it has been discovered that the lightly- to moderately-crosslinked PVP increases the viscosity of these compositions, stabilizing the viscosity and pH of these formulations that historically have proved difficult to thicken and stabilize. Lightly- to moderately-crosslinked PVP creates elegant, smooth, thickened compositions even at a pH as low as 1.3, a performance that is essentially unmatched by other thickeners.

Additionally, the invention relates to the use of these thickened compositions to deliver the acid to the skin, scalp, feet, or lips of a mammal, preferably man. Even more surprising, it has been discovered that the use of such thickened acidic compositions reduce irritation and sting discomfort compared to an equivalent formulation not having the lightly- to moderately-crosslinked PVP.

Due to the inherent complexity in these compositions, their ingredients, product forms, and uses, it will be appreciated that definitions of terms will help describe preferred embodiments of the invention.

The term *personal care compositions* (*or formulations*) refer to compositions intended for topical use on a mammal, including, man, horses, cats, and dogs. These compositions include skin, hair, scalp, foot, or lip compositions, including those compositions that can be purchased with and without a doctor's prescription. These personal care compositions can provide any number of known benefits, such as: moisturize, prevent wrinkles, treat wrinkles, firm skin, treat blemishes, protect from ultraviolet radiation, protect from thermal damage, lighten skin color, remove dirt / soil / dead skin / blocked pores, and treat keratosis (e.g., corns, calluses, and warts). The personal care compositions also may comprise other active and non-active ingredients to assist in their benefit, delivery, spreadability, emolliency, film formation, stability, and/or thickening.

The term *lightly- to moderately-crosslinked PVP*, unless otherwise noted, specifically refers to polymer essentially consisting of lightly- to moderately-crosslinked poly(*N*-vinyl-2-pyrrolidone) having at least one of the following characteristics: (1) an aqueous swelling parameter defined by its gel volume from about 15 mL/g to about 300 mL/g, more preferably from about 15 mL/g to about 250 mL/g, and most preferably from about 15 mL/g to about 150 mL/g, or (2) a Brookfield viscosity of 5% lightly- to moderately-crosslinked PVP in a liquid carrier comprising water at 25°C of at least 2,000 cP, more preferably of at least about 5,000 cP, and most preferably of at least about 10,000 cP. Disclosure for these parameter ranges is provided in U.S. patent 5,073,614 and in Shih, J.S., *et al.* (1995). Synthesis methods for the lightly- to moderately-crosslinked PVP are disclosed in a number of references, including U.S. patents 5,073,614; 5,654,385; and 6,177,068. It is appreciated by a polymer scientist skilled in the art that the method of synthesis is immaterial, inasmuch as the produced polymer achieves at least one of the abovedefined parameters.

For example, U.S. patent '614 discloses different crosslinkers and crosslinker amounts that yield lightly- to moderately-crosslinked PVP suitable for the present invention. The effect of crosslinker amount on swell volume and viscosity is graphically presented in Shih, J.S., *et al.* (1995). Thus, the lightly- to moderately-crosslinked PVP may be produced by the precipitation polymerization method of the '614 patent, by the hydrogel method described in the '385 patent, or by the non-aqueous, heterogeneous polymerization method of the '068 patent. Certainly, other techniques are contemplated to synthesize this polymer, provided the product meets the aqueous swelling parameter and Brookfield viscosity requirements.

Final product viscosities may slightly vary for compositions containing lightly- to moderately-crosslinked PVP made by these different methods. Nonetheless, these variations are within the scope of the invention, as the lightly- to moderately-crosslinked PVPs thicken low pH compositions.

Unless otherwise specified, "lightly- to moderately-crosslinked PVP" does not refer to swellable but water-insoluble crosslinked PVP, such as the type sold into commercial trade under the trade name Polyclar^{®} by International Specialty Products, which differs from the abovedescribed lightly- to moderately-crosslinked PVP.

The term *viscosity* refers to the proportionality coefficient between shear stress and shear rate, and describes a composition's resistance to flow. Because viscosity is dependent on shear rate, specific measurement information (such as viscometer, flow apparatus/spindle, and shear rate) is required to properly define viscosity. As used herein, *viscosity* refers to the proportionality coefficient determined from low shear rate, rotational flow, especially the viscosity measured by the Brookfield LVT and Brookfield RVT viscometers operating at 10 revolutions per minute (rpm) at 25°C. References describing the Brookfield measurement of viscosities include the following, each of which is hereby incorporated in its entirety by reference: Thibodeau, L., "Measuring viscosity of pastes," American Laboratory News, June 2004; McGregor, R.G., "Shelf life: does viscosity matter?" Pharmaceutical Online, October 31, 2007; and McGregor, R.G., "When ointments disappoint, the viscosity story," Brookfield Engineering brochure.

The term *sub-formulation* refers to a composition having two or more ingredients that is first prepared and then later blended with other ingredients as necessary. For example, sub-formulations may be made containing thickening agent(s) and liquid carrier(s) [which may or may not be solvents for the thickening agent(s)] with or without additional ingredients, and then divided into specific lots for use in specific formulation(s) at a later time.

The term *topical* refers to any external parts of a mammal, such as man, horses, cats, and dogs, and especially man, and includes skin, hair, scalp, lips, and feet.

The term low *pH* refers to a pH of 6 or less.

The term *very low pHrefers* to a pH of 4 or less.

The term *extremely low pH refers* to a pH of 2 or less.

### First embodiment of the invention

In a first embodiment of the invention, topical compositions are provided that have at least one personal care acid or at least one pharmaceutical acid, and lightly- to moderately-crosslinked PVP as defined in claim 1. In these compositions the lightly- to moderately-crosslinked PVP functions, in part, as a thickener, especially to increase the low shear viscosity. It is surprising that lightly- to moderately-crosslinked PVP effectively thickens low pH, very low pH, and extremely low pH personal care and pharmaceutical compositions, with results that are essentially unmatched by existing thickeners.

By virtue of having at the least one personal care or pharmaceutical acid, these topical compositions have a pH of less than 4, and more preferably, are very low pH compositions. In especially preferred embodiments, these compositions have an extremely low pH. Generally speaking, very low pH and extremely low pH are of greatest interest to the invention, as these compositions have proved most problematic to thicken. As it will be discussed in greater detail separately, the use of acidic topical compositions thickened with lightly- to moderately-crosslinked PVP has been discovered to produce less skin irritation and sting than identical formulations without lightly- to moderately-crosslinked PVP.

A broad selection of personal care acid and pharmaceutical acid compositions may be successfully thickened as described herein. Hydroxy acid formulations most frequently exhibit acidic pHs that are difficult to thicken and stabilize. Hydroxy acids can be divided into four subfamilies: alpha hydroxy acids, beta hydroxy acids, alpha and beta hydroxy acids, and polyhydroxy acids.

Alpha hydroxy acids are frequently employed in skin lotions and the like, as they are among the most useful exfoliation agents. By definition, alpha hydroxy acids possess a carboxylic acid group with a hydroxyl group on the adjacent carbon atom. Both naturally occurring and synthetic alpha hydroxy acids are known and suitable for use. Examples of alpha hydroxy acids include, without limitation: alpha hydroxyethanoic acid, alpha hydroxyoctanoic acid, alpha hydroxycaprylic acid, ascorbic acid, adipic acid, caprylic acid, capric acid, glycolic acid, lactic acid, lauric acid, mandelic acid, myristic acid, palmitic acid, stearic acid, linoleic acid, linolenic acid, ricinoleic acid, oleic acid, tartaric acid, elaidic acid, and erucic acid.

Most preferred are alpha hydroxy acids that exhibit high epidermis penetration so that they may exert a maximum effect on the underlying dermis layer. Thus, the most effective alpha hydroxy acids are those of small molecular weight, such as glycolic acid and lactic acid. This preference, however, is not to say that the invention does not work in thickening higher molecular weight acids. Rather, this preference merely recognizes a special class of hydroxy acids that are used in many personal care and pharmaceutical compositions.

Beta hydroxy acids find utility in the invention and in skin care products due to their ability to penetrate the epidermis and activity in the dermal layer. Beta hydroxy acids are those molecules having a carboxylic acid group and a hydroxyl group separated by two carbon atoms. Again, both naturally occurring and synthetic beta hydroxy acids are known and may be used in the invention's compositions. Specific examples of beta hydroxy acids include, but are not limited to: beta hydroxybutanoic acid, tropic acid, trethocanic acid, salicylic acid, and 5-(*n*-octanoyl) salicylic acid.

Also for use in the thickened topical compositions are alpha beta hydroxy acids. As the same suggests, these acids contain at least one alpha hydroxy acid group and one beta hydroxy acid group, Examples of alpha beta hydroxy acids include: malic acid, citric acid, and tartaric acid.

A final member of the hydroxy acid family is the polyhydroxy acid, which, as the name suggests, are molecules having at least one carboxylic acid functional group and more than 1 hydroxyl group. Polyhydroxy acids also may be naturally occurring or synthetically manufacturered, and have a higher molecular weight than glycolic acid or lactic acid. As a result, polyhydroxy acids are less penetrating than these two alpha hydroxy acids, and, as a result, provide gentler skin effects, typically with reduced irritation. Examples of suitable polyhydroxy acids include lactobionic acid, galactose, and gluconic acid.

Non-hydroxy acids that may be used are: aminosulphonic compounds, (*N*-2-hydroxyethyl) piperazine-*N'*-2-ethanesulphonic acid; 2-oxothiazoline-4-carboxylic acid (procysteine), pyruvic acid, trichloroacetic acid, etidronic acid, dioic acid, azelaic acid, their salts, esters and derivatives, and blends thereof.

In order to achieve desired product performance, mixtures of different acids also may be thickened, as well as combinations of acids and the corresponding salts. Suitable such salts include the alkali metal salts of phosphoric and sulphuric acids, e.g. potassium biphosphate and sodium bisulphate.

The thickened topical compositions of the invention may be used where ever acidic personal care and acidic pharmaceutical preparations find utility. Accordingly, the amount of lightly- to moderately-crosslinked PVP in the composition depends on a variety of parameters, including the amount and type of acid(s), other ingredients, and the desired product form, delivery, and consumer "thickness" acceptance. For example, the thickened compositions may be an anti-aging cream, a lotion for skin blemishes, a smoothing lotion, a moisturizing composition, a skin lightening treatment, a shampoo, or a cream for use around the eyes or mouth. In these formulations the amount of lightly- to moderately-crosslinked PVP may vary from about 0.1% to about 10% (w/w) of the total formulation. More typically, however, the amount of lightly- to moderately-crosslinked PVP varies from about 1% to about 6% (w/w) of the total formulation. As illustrated in Examples 2-6, thickened acid systems containing from 43% to 71% glycolic acid were effectively thickened to viscosities ranging from 15,000 cP to 37,000 cP with the addition of 4.5% lightly- to moderately-crosslinked PVP.

At these addition levels of lightly- to moderately-crosslinked PVP, the thickened low pH compositions typically have a Brookfield viscosity, as measured at 10 rpm and 25°C using an appropriate spindle (e.g., T-C or T-E), from about 1,000 cP to about 100,000 cP. (Of course, the product Brookfield viscosity depends on the panoply of factors outlined in the preceding paragraph.) More preferably, based on the contemplated product forms, the compositions have a Brookfield viscosity from about 10,000 cP to 50,000 cP.

Because of the stabilized viscosity and pH provided by lightly- to moderately-crosslinked PVP in these low pH formulations, compositions comprising this thickener may be a sub-formulation or a complete formulation. Considering the challenges facing production scheduling, batch preparation, and formulation changes, for example, it may be advantageous to prepare a sub-formulation batch having the lightly- to moderately-crosslinked PVP, and then use portions of it at some later time to prepare one or more final formulations. Alternatively, a complete formulation with the lightly- to moderately-crosslinked PVP may be made at essentially in one batch. The compositions of Examples 2-6 may be viewed as examples of sub-formulations if they are not desired as stand-along gel preparations (e.g., for skin care).

It was mentioned earlier that the amount of lightly- to moderately-crosslinked PVP in the thickened, acidic formulation depends on a number of factors, including the desired product form. The compositions do not produce "pilling" (incompatibilities and/or phase separations/agglomeration resulting in lumps) nor impart a stringy texture to the composition even at extremely low pH. This relationship between lightly- to moderately-crosslinked PVP and viscosity cannot be overstated, as thickeners generally are not known for such low pH systems.

The thickening additive compositions in accordance with this disclosure can be easily prepared by conventional methods known to persons of ordinary skill in the art, employing methods such as, simple mixing, blending, and homogenization using physical means or heat blending.

### Second embodiment of the invention

In a second embodiment of the invention, the thickened topical compositions are used to deliver the personal care and/or pharmaceutical acid(s) to the skin, hair, scalp, foot, or lip of a mammal in need of treatment.

As an extension of this use, it has been discovered that the use of these thickened topical compositions reduce the discomfort of irritation and sting compared to an equivalent formulation without lightly- to moderately-crosslinked PVP. The merit of this claim was provided from three independent, third-party clinical laboratory evaluations, as discussed in Examples 10-12. Without being bound to theory, one school of thought is that lightly- to moderately-crosslinked PVP in these formulas creates a gel network with the acid(s), moderates its release, and thus makes these compositions gentler on skin.

Because irritation/sting was evaluated using the simple formulas of Examples 10-12, it will be appreciated by one skilled in the art that significant formulation development may be pursued to maximize the composition and use benefits embraced by this invention. For example, products may be formulated with exfoliation, firming, moisturizing, and/or dermal perfusion effect(s) comparable to existing products (without lightly- to moderately-crosslinked PVP), but which reduce or eliminate irritation and/or sting. Such products may be found to be exceedingly gentle even on the most sensitive of skin.

Alternatively, products can be formulated that maintain the level of irritation and/or sting of current products (without lightly- to moderately-crosslinked PVP), but which provide greater exfoliation, firming, moisturizing, and/or dermal perfusion effect(s). These products may be aimed at enhanced-performance product lines, or compositions intended to be used under the care of a physician.

### Optional: Additional formulation ingredients and adjutants

Due to the requirements of end performance, it is expected that the topical compositions of this invention will be used together with other additives to further enhance the properties of the finished product. Such ingredients may be incorporated without altering the scope of the current invention, and may be included in order to produce the necessary products.

These topical formulations inevitably have a liquid or liquid-like carrier that aides to distribute, disperse, and/or dissolve the formulation ingredients, including the lightly- to moderately-crosslinked PVP. Selection of these carriers is not limited, inasmuch as the formulations have at least one personal care acid or at least one pharmaceutical acid, and examples of liquid carriers include water, alcohols, oils, esters, and blends thereof.

The composition of the invention also can contain one or more additional additives chosen from conditioning agents, protecting agents, such as, for example, hydrosoluble, antiradical agents, antioxidants, vitamins, ultraviolet absorbers, and pro-vitamins, fixing agents, oxidizing agents, reducing agents, dyes, cleansing agents, anionic, cationic, nonionic and amphoteric surfactants, thickeners, perfumes, pearlizing agents, stabilizers, pH adjusters, filters, preservatives, cationic and nonionic polyether associative polyurethanes, polymers other than the cationic polymer described herein, vegetable oils, mineral oils, synthetic oils, polyols such as glycols and glycerol, silicones, aliphatic alcohols, colorants, bleaching agents, highlighting agents and sequestrants. These additives are present in the composition according to the invention in proportions that may range from 0% to 20% by weight in relation to the total weight of the composition. The precise amount of each additive may be easily determined by an expert in the field according to its nature and its function.

When the final product aims to protect the user from ultraviolet radiation, it may be desirable to include one or more UV absorbers. In this context, the terms *ultraviolet* and *UV* mean electromagnetic radiation, especially solar electromagnetic radiation, with a wavelength from about 100 nm to about 400 nm, and includes the UV-A, UV-B, and UV-C subclassifications of such radiation. The term *UV*-*A* means ultraviolet electromagnetic radiation with a wavelength from about 320 nm to about 400 nm, and includes UV-A1 (from about 340 nm to about 400 nm) and UV-A2 (from about 320 nm to about 340 nm).
The term *UV-B* means ultraviolet electromagnetic radiation with a wavelength from about 290 nm to about 320 nm. The term *UV*-*C* means ultraviolet electromagnetic radiation with a wavelength from about 200 nm to about 290 nm. Finally, the term *UV absorber* means any entity that absorbs, scatters, and/or reflects any wavelength of UV radiation.

Suitable UV absorbers that may be included in the topical compositions and uses of the invention most likely will depend on local regulations. Because the rules governing the names and usage levels evolve over time, it is impossible to include every UV absorber that may be used with the invention. Typical UV absorbers include, without limitation: octyl salicylate; pentyl dimethyl PABA; octyl dimethyl PABA; benzophenone-1; benzophenone-6; 2-(2H-benzotriazole-2-yl)-4,6-di-*tert*-pentylphenol; ethyl-2-cyano-3,3-diphenylacrylate; homomenthyl salicylate; bis-ethylhexyloxyphenol methoxyphenyl triazine; methyl-(1,2,2,6,6-pentamethyl-4-piperidyl)-sebacate; 2-(2H-benzotriazole-2-yl)-4-methylphenol; diethylhexyl butamido triazone; amyl dimethyl PABA; 4,6-bis(octylthiomethyl)-*o*-cresol; CAS number 65447-77-0; red petroleum; ethylhexyl triazone; octocrylene; isoamyl-*p*-methoxycinnamate; drometrizole; titanium dioxide; 2,4-di-*tert*-butyl-6-(5-chloro-2H-benzotriazole-2-yl)-phenol; 2-hydroxy-4-octyloxybenzophenone; benzophenone-2; diisopropyl methylcinnamate; PEG-25 PABA; 2-(1,1-dimethylethyl)-6-[[3-(1,1-demethylethyl)-2-hydroxy-5-methylphenyl]methyl-4-methylphenyl acrylate; drometrizole trisiloxane; menthyl anthranilate; butyl methoxydibenzoylmethane; 2-ethoxyethyl *p*-methoxycinnatnate; benzylidene camphor sulfonic acid; dimethoxyphenyl-[1-(3,4)]-4,4-dimethyl 1,3-pentanedione; zinc oxide; *N*,*N*'-hexane-1,6-diylbis[3-(3,5-di-*tert*-butyl-4-hydroxyphenylpropionamide)]; pentaerythritol tetrakis[3-(3,5-di-*tert*-butyl-4-hydroxyphenyl)propionate]; 2,6-di-*tert*-butyl-4-[4,6-bis(octylthio)-1,3,5-triazin-2-ylamino] phenol; 2-(2H-benzotriazole-2-yl)-4,6-bis(1-methyl-1-phenylethyl)phenol; trolamine salicylate; diethylanolamine *p*-methoxycinnarnate; polysilicone-15; CAS number 152261-33-1; 4-methylbenzylidene camphor; bisoctrizole; *N*-phenyl-benzenamine; reaction products with 2,4,4-trimethylpentene; sulisobenzone; (2-ethylhexyl)-2-cyano-3,3-diphenylaciylate; digalloyl trioleate; polyacrylamido methylbenzylidene camphor; glyceryl ethylhexanoate dimethoxycinnamate; 1,3-bis-[(2'-cyano-3',3'-diphenylacryloyl)oxy]-2,2-bis-{[(2'-cyano-bis-(2,2,6,6-tehamethyl-4-piperidyl)-sebacate; benzophenone-5; 1,3,5-tris(3,5-di-*tert*-butyl-4-hydroxybenzyl)-1,3,5-triazine-2,4,6(1H,3H,5H)-trione; hexamethylendiamine; benzophenone-8; ethyl-4-bis(hydroxypropyl) aminobenzoate; 6-*tert*-butyl-2-(5-chloro-2H-benzotriazole-2-yl)-4-methylphenol; *p*-aminabenzoic acid; 3,3',3",5,5',5"-hexa-*tert*-butyl-α-α'-α"-(mesitylenc-2,4,6-triyl)tri-*p*-cresol; lawsone with dihydroxyacetone; benzophenone-9; benzophenone-4; ethylhexyl dimethoxy benzylidene dioxoimidazoline propionate; *N,N'*-bisformyl-N,N'-bis-(2,2,6,6-tetramethyl-4-piperidinyl)-; 3-benzylidene camphor; terephthalylidene dicamphor sulfonic acid; camphor benzalkonium methosulfate; bisdisulizole disodium; etocrylene; ferulic acid; 2-(2H-benzotriazole-2-yl)-4-(1,1,3,3-tetramethylbutyl)-phenol; 4,6-bis(dodecylthiomethyl)-o-cresol; β-2-glucopyranoxy propyl hydroxy benzophenone; phenylbenzimidazole sulfonic acid; benzophenone-3; diethylamine hydroxybenzoyl hexylbenzoate; 3',3'-diphenylacryloyl)oxy]methyl}-propane; ethylhexyl *p*-methoxycinnamate, and blends thereof.

For example, the compositions according to the invention may be used to moisturize, soothe, retain moisture, and/or smooth skin, especially skin of the hands, elbows, and feet, and around the eyes and mouth. Highly preferred are thickened formulations that are non-greasy, such as lotions having glycerin, caprylic/capric triglycerides, hydrogenated cocoglycerides, and/or one or more vegetable oils (e.g., helianthus oil, soybean oil, linseed oil, and olive oil).

Any known conditioning agent is useful in the personal care compositions of this invention. Conditioning agents function to improve the cosmetic properties of the hair, particularly softness, thickening, untangling, feel, and static electricity and may be in liquid, semi-solid, or solid form such as oils, waxes, or gums. Similarly, any known skin altering agent is useful in the compositions of this invention. Preferred conditioning agents include cationic polymers, cationic surfactants and cationic silicones.

Conditioning agents may be chosen from synthesis oils, mineral oils, vegetable oils, fluorinated or perfluorinated oils, natural or synthetic waxes, silicones, cationic polymers, proteins and hydrolyzed proteins, ceramide type compounds, cationic surfactants, fatty amines, fatty acids and their derivatives, as well as mixtures of these different compounds.

The synthesis oils include polyolefins, *e.g.,* poly-α-olefins such as polybutenes, polyisobutenes and polydecenes. The polyolefins can be hydrogenated.

The mineral oils suitable for use in the compositions of the invention include hexadecane and oil of paraffin.

A list of suitable animal and vegetable oils comprises sunflower, corn, soy, avocado, jojoba, squash, raisin seed, sesame seed, walnut oils, fish oils, glycerol tricaprocaprylate, Purcellin oil or liquid jojoba, and blends thereof.

Suitable natural or synthetic oils include eucalyptus, lavender, vetiver, litsea cubeba, lemon, sandalwood, rosemary, chamomile, savory, nutmeg, cinnamon, hyssop, caraway, orange, geranium, cade, and bergamot.

Suitable natural and synthetic waxes include carnauba wax, candelila wax, alfa wax, paraffin wax, ozokerite wax, vegetable waxes such as olive wax, rice wax, hydrogenated jojoba wax, absolute flower waxes such as black currant flower wax, animal waxes such as bees wax, modified bees wax (cerabellina), marine waxes and polyolefin waxes such as polyethylene wax, and blends thereof.

The cationic polymers that may be used as a conditioning agent according to the invention are those known to improve the cosmetic properties of hair treated by detergent compositions. The expression "cationic polymer" as used herein, indicates any polymer containing cationic groups and/or ionizable groups in cationic groups. The cationic polymers used generally have a molecular weight the average number of which falls between about 500 Da and 5,000,000 Da and preferably between 1000 Da and 3,000,000 Da.

The preferred cationic polymers are chosen from among those containing units including primary, secondary, tertiary, and/or quaternary amine groups that may either form part of the main polymer chain or a side chain.

Useful cationic polymers include known polyamine, polyaminoamide, and quaternary polyammonium types of polymers, such as:
(1) homopolymers and copolymers derived from acrylic or methacrylic esters or amides. The copolymers can contain one or more units derived from acrylamides, methacrylamides, diacetone acrylamides, acrylamides and methacrylamides, acrylic or methacrylic acids or their esters, vinyllactams such as vinyl pyrrolidone or vinyl caprolactam, and vinyl esters. Specific examples include: copolymers of acrylamide and dimethyl amino ethyl methacrylate quaternized with dimethyl sulfate or with an alkyl halide; copolymers of acrylamide and methacryloyl oxyethyl trimethyl ammonium chloride; the copolymer of acrylamide and methacryloyl oxyethyl trimethyl ammonium methosulfate; copolymers of vinyl pyrrolidone/dialkylaminoalkyl acrylate or methacrylate, optionally quaternized, such as the products sold under the name Gafquat^{®} by International Specialty Products; the dimethyl amino ethyl methacrylate/vinyl caprolactam/vinyl pyrrolidone terpolymers, such as the product sold under the name Gaffix^{®} VC 713 by International Specialty Products; the vinyl pyrrolidone/methacrylamidopropyl dimethylamine copolymer, marketed under the name Styleze^{®} CC 10 by International Specialty Products; and the vinyl pyrrolidone/quaternized dimethyl amino propyl methacrylamide copolymers such as the product sold under the name Gafquat^{®} HS 100 by International Specialty Products (Wayne, NJ).
(2) derivatives of cellulose ethers containing quaternary ammonium groups, such as hydroxy ethyl cellulose quaternary ammonium that has reacted with an epoxide substituted by a trimethyl ammonium group.
(3) derivatives of cationic cellulose such as cellulose copolymers or derivatives of cellulose grafted with a hydrosoluble quaternary ammonium monomer, as described in U.S. patent 4,131,576, such as the hydroxy alkyl cellulose, and the hydroxymethyl-, hydroxyethyl- or hydroxypropyl- cellulose grafted with a salt of methacryloyl ethyl trimethyl ammonium, methacrylamidopropyl trimethyl ammonium, or dimethyl diallyl ammonium.
(4) cationic polysaccharides such as described in U.S. patents 3,589,578 and 4,031,307, guar gums containing cationic trialkyl ammonium groups and guar gums modified by a salt, *e.g.,* chloride of 2,3-epoxy propyl trimethyl ammonium.
(5) polymers composed of piperazinyl units and alkylene or hydroxy alkylene divalent radicals with straight or branched chains, possibly interrupted by atoms of oxygen, sulfur, nitrogen, or by aromatic or heterocyclic cycles, as well as the products of the oxidation and/or quaternization of such polymers.
(6) water-soluble polyamino amides prepared by polycondensation of an acid compound with a polyamine. These polyamino amides may be reticulated.
(7) derivatives of polyamino amides resulting from the condensation of polyalcoylene polyamines with polycarboxylic acids followed by alcoylation by bi-functional agents.
(8) polymers obtained by reaction of a polyalkylene polyamine containing two primary amine groups and at least one secondary amine group with a dioxycarboxylic acid chosen from among diglycolic acid and saturated dicarboxylic aliphatic acids having 3 to 8 atoms of carbon. Such polymers are described in U.S. Patents 3,227,615 and 2,961,347.
(9) the cyclopolymers of alkyl dialyl amine or dialkyl diallyl ammonium such as the homopolymer of dimethyl diallyl ammonium chloride and copolymers of diallyl dimethyl ammonium chloride and acrylamide.
(10) quaternary diammonium polymers such as hexadimethrine chloride.
(11) quaternary polyammonium polymers, including, for example, Mirapal^{®} A 15, Mirapol^{®} AD1, Mirapol^{®} AZ1, and Mirapol^{®} 175 products sold by Miranol.
(12) the quaternary polymers of vinyl pyrrolidone and vinyl imidazole such as the products sold under the names Luviquat^{®} FC 905, FC 550, and FC 370 by BASF Corporation.
(13) quaternary polyamines.
(14) reticulated polymers known in the art.

Other cationic polymers that may be used within the context of the invention are cationic proteins or hydrolyzed cationic proteins, polyalkyleneimines such as polyethyleneimines, polymers containing vinyl pyridine or vinyl pyridinium units, condensates of polyamines and epichlorhydrins, quaternary polyurethanes, and derivatives of chitin.

Preferred cationic polymers are derivatives of quaternary cellulose ethers, the homopolymers and copolymers of dimethyl diallyl ammonium chloride, quaternary polymers of vinyl pyrrolidone and vinyl imidazole, and mixtures thereof.

The conditioning agent can be any silicone known by those skilled in the art to be useful as a conditioning agent. The silicones suitable for use according to the invention include polyorganosiloxanes that are insoluble in the composition. The silicones may be present in the form of oils, waxes, resins, or gums. They may be volatile or non-volatile. The silicones can be selected from polyalkyl siloxanes, polyaryl siloxanes, polyalkyl aryl siloxanes, silicone gums and resins, and polyorgano siloxanes modified by organofunctional groups, and mixtures thereof.

Suitable polyalkyl siloxanes include polydimethyl siloxanes with terminal trimethyl silyl groups or terminal dimethyl silanol groups (dimethiconol) and polyalkyl (C₁-C₂₀) siloxanes.

Suitable polyalkyl aryl siloxanes include polydimethyl methyl phenyl siloxanes and polydimethyl diphenyl siloxanes, linear or branched.

The silicone gums suitable for use herein include polydiorganosiloxanes preferably having a number-average molecular weight between 200,000 Da and 1,000,000, Da used alone or mixed with a solvent. Examples include polymethyl siloxane, polydimethyl siloxane/methyl vinyl siloxane gums, polydimethyl siloxane/diphenyl siloxane, polydimethyl siloxane/phenyl methyl siloxane and polydimethyl siloxane/diphenyl siloxane/methyl vinyl siloxane.

Suitable silicone resins include silicones with a dimethyl/trimethyl siloxane structure and resins of the trimethyl siloxysilicate type.

The organo-modified silicones suitable for use in the invention include silicones such as those previously defined and containing one or more organofunctional groups attached by means of a hydrocarbon radical and grafted siliconated polymers. Particularly preferred are amino functional silicones.

The silicones may be used in the form of emulsions, nano-emulsions, or micro-emulsions.

The conditioning agent can be a protein or hydrolyzed cationic or non-cationic protein. Examples of these compounds include hydrolyzed collagens having triethyl ammonium groups, hydrolyzed collagens having trimethyl ammonium and trimethyl stearyl ammonium chloride groups, hydrolyzed animal proteins having trimethyl benzyl ammonium groups (benzyltrimonium hydrolyzed animal protein), hydrolyzed proteins having groups of quaternary ammonium on the polypeptide chain, including at least one C₁-C₁₈ alkyl.

Hydrolyzed proteins include Croquat L, in which the quaternary ammonium groups include a C₁₂ alkyl group, Croquat M, in which the quaternary ammonium groups include C₁₀-C₁₈ alkyl groups, Croquat S in which the quaternary ammonium groups include a C₁₈ alkyl group and Crotein Q in which the quaternary ammonium groups include at least one C₁-C₁₈ alkyl group. These products are sold by Croda.

The conditioning agent can comprise quaternized vegetable proteins such as wheat, corn, or soy proteins such as cocodimonium hydrolyzed wheat protein, laurdimonium hydrolyzed wheat protein and steardimonium hydrolyzed wheat protein, 2-N-stearoyl amino-octadecane-1,3-diol, 2-N-behenoyl amino-octadecane-1,3-diol, 2-*N-*[2-hydroxy-palmitoyl)-amino-octadecane-1,3-diol, 2-*N-*stearoyl amino-octadecane-1,3,4-triol, *N*-stearoyl phytosphingosine, 2-*N*-palmitoyl amino-hexadecane-1,3-diol, bis-(*N-*hydroxy ethyl N-cetyl) malonamide, *N*-(2-hydroxy ethyl)-*N-*(3-cetoxyl-2-hydroxy propyl) amide of cetylic acid, *N*-docosanoyl *N*-methyl-D-glucamine and mixtures of such compounds.

The conditioning agent can be a cationic surfactant such as a salt of a primary, secondary, or tertiary fatty amine, optionally polyoxyalkylenated, a quaternary ammonium salt, a derivative of imadazoline, or an amine oxide. Suitable examples include mono-, di-, or tri- alkyl quaternary ammonium compounds with a counterion such as a chloride, methosulfate, tosylate, etc. including, but not limited to, cetrimonium chloride, dicetyldimonium chloride, behentrimonium methosulfate, and the like. The presence of a quaternary ammonium compound in conjunction with the polymer described above reduces static and enhances combing of hair in the dry state. The polymer also enhances the deposition of the quaternary ammonium compound onto the hair substrate thus enhancing the conditioning effect of hair.

The conditioning agent can be any fatty amine known to be useful as a conditioning agent; *e.g.* dodecyl, cetyl or stearyl amines, such as stearamidopropyl dimethylamine.

The conditioning agent can be a fatty acid or derivatives thereof known to be useful as conditioning agents. Suitable fatty acids include myristic acid, palmitic acid, stearic acid, behenic acid, oleic acid, linoleic acid, and isostearic acid. The derivatives of fatty acids include carboxylic ester acids including mono-, di-, tri- and tetra- carboxylic acids.

The conditioning agent can be a fluorinated or perfluorinated oil. The fluoridated oils may also be fluorocarbons such as fluoramines, *e.g.,* perfluorotributylamine, fluoridated hydrocarbons, such as perfluorodecahydronaphthalene, fluoroesters, and fluoroethers.

Of course, mixtures of two or more conditioning agents can be used.

The conditioning agent or agents can be present in an amount of 0.001% to 20%, preferably from 0.01% to 10%, and even more preferably from 0.1% to 3% by weight based on the total weight of the final composition.

The antioxidants or antiradical agents can be selected from phenols such as BHA (*tert-*butyl-4-hydroxy anisole), BHT (2,6-di-*tert*-butyl-p-cresol), TBHQ (*tert*-butyl hydroquinone), polyphenols such as proanthocyanodic oligomers, flavonoids, hindered amines such as tetra amino piperidine, erythorbic acid, polyamines such as spermine, cysteine, glutathione, superoxide dismutase, and lactoferrin.

The vitamins can be selected from ascorbic acid (vitamin C), vitamin E, vitamin E acetate, vitamin E phosphate, B vitamins such as B3 and B5, niacin, vitamin A, and derivatives thereof. The provitamins can be selected from panthenol and retinol.

The protecting agent can be present in an amount 0.001% to 20% by weight, preferably from 0.01% to 10% by weight, and more preferably 0.1 to 5% by weight of the total weight of the final composition.

In addition, the compositions according to the invention advantageously include at least one surfactant, which can be present in an amount of 0.1% and 60% preferably 1% and 40%, and more preferably 5% and 30% by weight based on the total weight of the composition. The surfactant may be chosen from among anionic, amphoteric, or non-ionic surfactants, or mixtures of them known to be useful in personal care compositions.

Additional thickeners or viscosity increasing agents may be included in the composition of the invention, such as: Acetamide MEA; acrylamide/ethalkonium chloride acrylate copolymer; acrylamidelethyltrimonium chloride acrylate/ethalkonium chloride acrylate copolymer; acrylamides copolymer; acrylamide/sodium acrylate copolymer; acrylamide/sodium acryloyldimethyltaurate copolymer; acrylates/acetoacetoxyethyl methacrylate copolymer; acrylates/beheneth-25 methacrylate copolymer; acrylates/C₁₀-C₃₀ alkyl acrylate crosspolymer; acrylates/ceteth-20 itaconate copolymer; acrylates/ceteth-20 methacrylate copolymer; acrylates/laureth-25 methacrylate copolymer; acrylates/palmeth-25 acrylate copolymer; acrylates/palmeth-25 itaconate copolymer; acrylates/steareth-50 acrylate copolymer; acrylates/steareth-20 itaconate copolymer; acrylates/steareth-20 methacrylate copolymer; acrylates/stearyl methacrylate copolymer; acrylates/vinyl isodecanoate crosspolymer; acrylic acidlacrylonitrogens copolymer; adipic acid/methyl DEA crosspolymer; agar; agarose; alcaligenes polysaccharides; algin; alginic acid; almondamide DEA; almondamidopropyl betaine; aluminum/magnesium hydroxide stearate; ammonium acrylates/acrylonitrogens copolymer; ammonium acrylates copolymer; ammonium acryloyldimethyltaurate/vinyl formamide copolymer; ammonium acryloyldimethyltaurate/VP copolymer; ammonium alginate; ammonium chloride; ammonium polyacryloyldimethyl taurate; ammonium sulfate; amylopectin; apricotamide DEA; apricotamidopropyl betaine; arachidyl alcohol; arachidyl glycol; arachis hypogaea (peanut) flour; ascorbyl methylsilanol pectinate; astragalus gummifer gum; attapulgite; avena sativa (oat) kernel flour; avocadamide DEA; avocadamidopropyl betaine; azelamide MEA; babassuamide DEA; babassuamide MEA; babassuamidopropyl betaine; behenamide DEA; behenamide MEA; behenamidopropyl betaine; behenyl betaine; bentonite; butoxy chitosan; caesalpinia spinosa gum; calcium alginate; calcium carboxymethyl cellulose; calcium carrageenan; calcium chloride; calcium potassium carbomer; calcium starch octenylsuccinate; C20-40 alkyl stearate; canolamidopropyl betaine; capramide DEA; capryl/capramidopropyl betaine; carbomer; carboxybutyl chitosan; carboxymethyl cellulose acetate butyrate; carboxymethyl chitin; carboxymethyl chitosan; carboxymethyl dextran; carboxymethyl hydroxyethylcellulose; carboxymethyl hydroxypropyl guar; carnitine; cellulose acetate propionate carboxylate; cellulose gum; ceratonia siliqua gum; cetearyl alcohol; cetyl alcohol; cetyl babassuate; cetyl betaine; cetyl glycol; cetyl hydroxyethylcellulose; chimyl alcohol; cholesterol/HDI/pullulan copolymer; cholesteryl hexyl dicarbamate pullulan; citrus aurantium dulcis (orange) peel extract; cocamide DEA; cocamide MEA; cocamide MIPA; cocamidoethyl betaine; cocamidopropyl betaine; cocamidopropyl hydroxysultaine; coco-betaine; coco-hydroxysultaine; coconut alcohol; coco/oleamidopropyl betaine; coco-Sultaine; cocoyl sarcosinamide DEA; cornamide/cocamide DEA; cornamide DEA; croscarmellose; crosslinked bacillus/glucose/sodium glutamate ferment; cyamopsis tetragonoloba (guar) gum; decyl alcohol; decyl betaine; dehydroxanthan gum; dextrin; dibenzylidene sorbitol; diethanolaminooleamide DEA; diglycol/CHDM/isophthalates/SIP copolymer; dihydroabietyl behenate; dihydrogenated tallow benzylmonium hectorite; dihydroxyaluminum aminoacetate; dimethicone/PEG-10 crosspolymer; dimethicone/PEG-15 crosspolymer; dimethicone propyl PG-betaine; dimethylacrylamide/acrylic acid/polystyrene ethyl methacrylate copolymer; dimethylacrylamide/sodium acryloyldimethyltaurate crosspolymer; disteareth-100 IPDI; DMAPA acrylates/acrylic acid/acrylonitrogens copolymer; erucamidopropyl hydroxysultaine; ethylene/sodium acrylate copolymer; gelatin; gellan gum; glyceryl alginate; glycine soja (soybean) flour; guar hydroxypropyltrimonium chloride; hectorite; hyaluronic acid; hydrated silica; hydrogenated potato starch; hydrogenated tallow; hydrogenated tallowamide DEA; hydrogenated tallow betaine; hydroxybutyl methylcellulose; hydroxyethyl acrylate/sodium acryloyldimethyl taurate copolymer; hydroxyethylcellulose; hydroxyethyl chitosan; hydroxyethyl ethylcellulose; hydroxyethyl stearamide-MIPA; hydroxylauryllhydroxymyristyl betaine; hydroxypropylcellulose; hydroxypropyl chitosan; hydroxypropyl ethylenediamine carbomer; hydroxypropyl guar; hydroxypropyl methylcellulose; hydroxypropyl methylcellulose stearoxy ether; hydroxypropyl starch; hydroxypropyl starch phosphate; hydroxypropyl xanthan gum; hydroxystearamide MEA; isobutylenelsodium maleate copolymer; isostearamide DEA; isostearamide MEA; isostearamide mIPA; isostearamidopropyl betaine; lactamide MEA; lanolinamide DEA; lauramide DEA; lauramide MEA; lauramide MIPA; lauramide/myristamide DEA; lauramidopropyl betaine; lauramidopropyl hydroxysultaine; laurimino bispropanediol; lauryl alcohol; lauryl betaine; lauryl hydroxysultaine; lauryl/myristyl glycol hydroxypropyl ether; lauryl sultaine; lecithinamide DEA; linoleamide DEA; linoleamide MEA; linoleamide MIPA; lithium magnesium silicate; lithium magnesium sodium silicate; macrocystis pyrifera (kelp); magnesium alginate; magnesium/aluminum/hydroxide/carbonate; magnesium aluminum silicate; magnesium silicate; magnesium trisilicate; methoxy PEG-22/dodecyl glycol copolymer; methylcellulose; methyl ethylcellulose; methyl hydroxyethylcellulose; microcrystalline cellulose; milkamidopropyl betaine; minkamide DEA; minkamidopropyl betaine; MIPA-myristate; montmorillonite; Moroccan lava clay; myristamide DEA; myristamide MEA; myristamide MIPA; myristamidopropyl betaine; myristamidopropyl hydroxysultaine; myristyl alcohol; myristyl betaine; natto gum; nonoxynyl hydroxyethylcellulose; oatamide MEA; oatamidopropyl betaine; octacosanyl glycol isostearate; octadecene/MA copolymer; oleamide DEA; oleamide MEA; oleamide MIPA; oleamidopropyl betaine; oleamidopropyl hydroxysultaine; oleyl betaine; olivamide DEA; olivamidopropyl betaine; oliveamide MEA; palmamide DEA; palmamide MEA; palmamide MIPA; palmamidopropyl betaine; palmitamide DEA; palmitamide MEA; palmitamidopropyl betaine; palm kernel alcohol; palm kemelamide DEA; palm kernelamide MEA; palm kernelamide MIPA; palm kernelamidopropyl betaine; peanutamide MEA; peanutamide MIPA; pectin; PEG-800; PEG-crosspolymer; PEG-150/decyl alcohol/SMDI copolymer; PEG-175 diisostearate; PEG-190 distearate; PEG-15 glyceryl tristearate; PEG-140 glyceryl tristearate; PEG-240/HDI copolymer bis-decyltetradeceth-20 ether; PEG-100/IPDI copolymer; PEG-180/laureth-50/TMMG copolymer; PEG-10/lauryl dimethicone crosspolymer; PEG-15/lauryl dimethicone crosspolymer; PEG-2M; PEG-5M; PEG-7M; PEG-9M; PEG-14M; PEG-20M; PEG-23M; PEG-25M; PEG-45M; PEG-65M; PEG-90M; PEG-115M; PEG-160M; PEG-180M; PEG-120 methyl glucose trioleate; PEG-180/octoxynol-40/TMMG copolymer; PEG-150 pentaerythrityl tetrastearate; PEG-4 rapeseedamide; PEG-150/stearyl alcohol/SMDI copolymer; phaseolus angularis seed powder; polianthes tuberosa extract; polyacrylate-3; polyacrylic acid; polycyclopentadiene; polyether-1; polyethylene/isopropyl maleate/MA copolyol; polyglyceryl-3 disiloxane dimethicone; polyglyceryl-3 polydimethylsiloxyethyl dimethicone; polymethacrylic acid; polyquaternium-52; polyvinyl alcohol; potassium alginate; potassium aluminum polyacrylate; potassium carbomer; potassium carrageenan; potassium chloride; potassium palmate; potassium polyacrylate; potassium sulfate; potato starch modified; PPG-2 cocamide; PPG-1 hydroxyethyl caprylamide; PPG-2 hydroxyethyl cocamide; PPG-2 hydroxyethyl coco/isostearamide; PPG-3 hydroxyethyl soyamide; PPG-14 laureth-60 hexyl dicarbamate; PPG-14 laureth-60 isophoryl dicarbamate; PPG-14 palmeth-60 hexyl dicarbamate; propylene glycol alginate; PVP/decene copolymer; PVP montmorillonite; pyrus cydonia seed; pyrus malus (apple) fiber; rhizobian gum; ricebranamide DEA; ricinoleamide DEA; ricinoleamide MEA; ricinoleamide MIPA; ricinoleamidopropyl betaine; ricinoleic acid/adipic acid/AEEA copolymer; rosa multiflora flower wax; sclerotium gum; sesamide DEA; sesamidopropyl betaine; sodium acrylate/acryloyldimethyl taurate copolymer; sodium acrylates/acrolein copolymer; sodium acrylates/acrylomtrogens copolymer; sodium acrylates copolymer; sodium acrylates crosspolymer; sodium acrylate/sodium acrylamidomethylpropane sulfonate copolymer; sodium acrylates/vinyl isodecanoate crosspolymer; sodium acrylate/vinyl alcohol copolymer; sodium carbomer; sodium carboxymethyl chitin; sodium carboxymethyl dextran; sodium carboxymethyl beta-glucan; sodium carboxymethyl starch; sodium carrageenan; sodium cellulose sulfate; sodium chloride; sodium cyclodextrin sulfate; sodium hydroxypropyl starch phosphate; sodium isooctylene/MA copolymer; sodium magnesium fluorosilicate; sodium oleate; sodium palmitate; sodium palm kemelate; sodium polyacrylate; sodium polyacrylate starch; sodium polyacryloyldimethyl taurate; sodium polygamma-glutamate; sodium polymethacrylate; sodium polystyrene sulfonate; sodium silicoaluminate; sodium starch octenylsuccinate; sodium stearate; sodium stearoxy PG-hydroxyethylcellulose sulfonate; sodium styrene/acrylates copolymer; sodium sulfate; sodium tallowate; sodium tauride acrylates/acrylic acid/acrylonitrogens copolymer; sodium tocopheryl phosphate; solanum tuberosum (potato) starch; soyamide DEA; soyamidopropyl betaine; starch/acrylates/acrylamide copolymer; starch hydroxypropyltrimonium chloride; stearamide AMP; stearamide DEA; stearamide DEA-distearate; stearamide DIBA-stearate; stearamide MEA; stearamide MEA-stearate; stearamide MIPA; stearamidopropyl betaine; steareth-60 cetyl ether; steareth-100/PEG-136/HDI copolymer; stearyl alcohol; stearyl betaine; sterculia urens gum; synthetic fluorphlogopite; tallamide DEA; tallow alcohol; tallowamide DEA; tallowamide MEA; tallowamidopropyl betaine; tallowamidopropyl hydroxysultaine; tallowamine oxide; tallow betaine; tallow dihydroxyethyl betaine; tamarindus indica seed gum; tapioca starch; TEA-alginate; TEA-carbomer; TEA-hydrochloride; trideceth-2 carboxamide MEA; tridecyl alcohol; triethylene glycol dibenzoate; trimethyl pentanol hydroxyethyl ether; triticum vulgare (wheat) germ powder; triticum vulgare (wheat) kernel flour; triticum vulgare (wheat) starch; tromethamine acrylates/acrylonitrogens copolymer; tromethamine magnesium aluminum silicate; undecyl alcohol; undecylenamide DEA; undecylenamide MEA; undecylenamidopropyl betaine; welan gum; wheat germamide DEA; wheat germatnidoprapyl betaine; xanthan gum; yeast beta-glucan; yeast polysaccharides and zea mays (corn) starch.

### Product forms

Acknowledging the many ways topical personal care and pharmaceutical compositions may be used, it is within the scope of the invention that the thickened compositions may have the form of a solution, a cream, an ointment, a lotion, an oil-in-water emulsion, a water-in-oil emulsion, a shampoo, a spray, a gel, a wash, a rinse, an aerosol, a suspension, a paste, a powder, a serum, or a mousse.

In other examples of the invention, thickened compositions may be used to wash and treat keratinous material such as hair, skin, eyelashes, eyebrows, fingernails, lips, and hairy skin, The compositions of the invention may also take the form of skin-washing compositions, and particularly in the form of solutions or gels for the bath or shower, or of make-up removal products.

The compositions according to the invention may also take the form of after-shampoo compositions, to be rinsed off or not, for permanents, straightening, waving, dyeing, or bleaching, or the form of rinse compositions to be applied before or after dyeing, bleaching, permanents, straightening, relaxing, waving or even between the two stages of a permanent or straightening process.

Examples of related compositions are disclosed in U.S. patents 5,599,800; 5,650,166; 5,916,549; and 6,812,192; U.S. patent application 2009/0317432; EP 556,660; 661,037; 661,038; 662,315; 676,194; 796,077; 970,682; 976383; 1,415,654; and 2,067,467; and WO 2005/032506;.

The compositions according to the invention can be detergent compositions such as shampoos, bath gels, and bubble baths. In this mode, the compositions will comprise water as a liquid carrier. The surfactant or surfactants that form the washing base may be chosen alone or in blends, from known anionic, amphoteric, or non-ionic surfactants. The quantity and quality of the washing base must be sufficient to impart a satisfactory foaming and/or detergent value to the final composition. The washing base can be from 4% to 50% by weight, preferably from 6% to 35% by weight, and even more preferentially from 8% to 25% by weight of the total weight of the final composition.

Cosmetic compositions according to the invention may, for example, be used as care and/or sun protection product for the face and/or the body having a consistency ranging from liquid to semiliquid (*e.g.,* milks, creams), and gels, creams, pastes, powders (including compacted powders), and wax-like compositions (*e.g.,* lip balms).

For compositions intended to protect the hair from UV radiation, suitable product forms include, but not limited to: conditioners, dispersions, emulsions, gels, lotions, mists, mousses, shampoos, and sprays.

The personal care active includes shampoo, body wash products, shaving cream, hand soap, bubble bath, bath gel, after-shave lotions, creams, moisturizers, sunscreens, liquid soaps, color cosmetics, acid peels, perms, hair color, sunless tanning and conditioners.

Due to the low pH of these topical compositions, they may be expected provide a skin exfoliation effect (also known as keratolysis). As such, these acidic formulations find use in treating wrinkles and dry skin. Other skin and scalp conditions that can be treated by these thickened, low pH compositions also are contemplated, for example, the use of thickened salicylic acid formulations for the treatment of various warts, corns, and calluses. Examples of wart-removal compositions include the following, each of which is incorporated herein its entirety by reference: U.S. patents 5,962,011 and 7,655,668; US patent application 2007/0280972; EP 1,002,530; and WO 2009/085890. Examples of skin lightening compositions and age-spot compositions include the following, U.S. 5,747,051; U.S. patent application 2008/0214669; EP 1028723; and WO 2004/073745.

The following examples are presented to illustrate specific embodiments of the present compositions and methods.

### EXAMPLES

### Example 1: Ascorbic acid and glycolic acid gels

Two formulations were prepared containing 10% ascorbic acid or 10% glycolic acid in water with 5% lightly- to moderately-crosslinked PVP (Table 1). Neither composition phase separated or coagulated, but rather both were smooth, low pH gels as indicated in Table 1.

**Table 1: Low pH glycolic acid and ascorbic acid gels of Example 1.**

| **active** | **liquid carrier** | **lightly- to moderately-crosslinked PVP** | **initial pH^{†}** | **viscosity*** |
|---|---|---|---|---|
| 10% ascorbic acid | water | 5% | 3.88 | 23,000 |
| 10% glycolic acid | water | 5% | 3.92 | 13,500 |

| | | | | |
|---|---|---|---|---|
| ^{†}pH was measured at 25°C. *Viscosity was measured using a Brookfield LVT viscometer with spindle T-E at 10 rpm and 25°C, | | | | |

### Examples 2-6: Thickened acidic systems having lightly- to moderately-crosslinked PVP

Five low pH compositions of the invention were made by blending between 4.5%-6.0% lightly- to moderately-crosslinked PVP, a personal care acid, and at least one liquid carrier (Table 2). The five preparations were smooth gels having a pH less than 3.0 and viscosities of 15,000 cP or more.

Thickened acidic systems such as these may represent stand-alone formulations. Alternatively, their pH and viscosity stability allows them to be treated as sub-formulations to be prepared in advance, and then to be added to other ingredients as necessary.

**Table 2: Thickened acidic systems of Examples 2-6**

| **ex.** | **ingredients** | **addition level (% w/w)** | **appearance** | **pH^{†}** | **viscosity *** |
|---|---|---|---|---|---|
| 2 | lightly- to moderately-crosslinked PVP | 4.5 | gel | 1.68 | 15,000 |
| | glycolic acid, (70% solution) | 43.0 | | | |
| | deionized water | 52.5 | | | |
| | *total* | 100.0 | | | |
| 3 | lightly- to moderately-crosslinked PVP | 6.0 | gel | 2.9 | 22,000 |
| | salicylic acid, USP | 10.0 | | | |
| | SD alcohol 40 | 84.0 | | | |
| | *total* | 100.0 | | | |
| 4 | lightly- to moderately-crosslinked PVP | 4.5 | gel | 1.32 | 30,000 |
| | glycolic acid, (70% solution) | 71.0 | | | |
| | deionized water | 24.5 | | | |
| | *total* | 100. | | | |
| 5 | lightly- to moderately-crosslinked PVP | 4.5 | gel | 1.35 | 35,000 |
| | glycolic acid, (70% solution) | 71.0 | | | |
| | deionized water | 14.5 | | | |
| | SD alcohol 40 | 10.0 | | | |
| | *total* | 100.0 | | | |
| 6 | lightly- to moderately-crosslinked PVP | 4.5 | gel | 1.45 | 37,000 |
| | glycolic acid (70% solution) | 71.0 | | | |
| | deionized water | 4.5 | | | |
| | SD alcohol 40 | 20.0 | | | |
| | *total* | 100.0 | | | |

| | | | | | |
|---|---|---|---|---|---|
| ^{†}pH was measured at 255°C. *Viscosity measured using a Brookfield LVT viscometer with spindle T-C at 10 rpm and 25°C. | | | | | |

### Example 7: Acne gel preparation

An acne gel preparation was made containing two active ingredients, 2% salicylic acid and 5% glycolic acid (Table 3). First, salicylic acid was dissolved in ethanol, to which water and glycolic then were added with mixing. The pH of this sub-formulation was adjusted to 4.2 using ammonium hydroxide solution. Then, lightly- to moderately crosslinked PVP was added followed by homogenization. To this thickened gel two emollients (Ceraphyl^{®} 41 and Lubrajel^{®} Oil) were added.

The preparation described above appeared as a gel, and the measured pH was 4. The viscosity, measured by a Brookfield RVT viscometer using spindle T-C at 10 rpm and 25°C was 24,000 cP.

**Table 3: Acne gel formulation of Example 7.**

| **ingredient** | **addition level (% w/w)** |
|---|---|
| Phase A | |
| water | 38.9 |
| salicylic acid | 2.0 |
| glycolic acid (70%) | 7.2 |
| ammonium hydroxide solution (28%-30%) | 1.4 |
| *total* | 49.5 |

| Phase B | |
|---|---|
| ethanol | 40.0 |
| lightly- to moderately-crosslinked PVP | 5.0 |
| *total* | 45.0 |

| Phase C | |
|---|---|
| Ceraphyl^{®} 41 | 3.0 |
| Lubrajel^{®} Oil | 2.5 |
| *total* | 5.5 |
| ***grand total*** | **100.0** |

### Example 8: Stability of acne gel preparation of Example 7

The acne gel of Example 7 was placed on stability testing at 5°C, 25°C, and 45°C to determine if viscosity or pH changed over time or after freeze / thaw cycles. Viscosity was measured using a Brookfield RVT viscometer with an T-C spindle at 10 rpm. Freeze / thaw cycles were defined as freezing overnight at -15°C, followed by next morning thaw at 25°C until the acne gel reached 25°C.

Measured viscosities at 5°C and 25°C were essentially constant over the 12 week test period (Figure 1). Storage at 45°C produced slightly increased viscosity, from an initial value of 24,000 cP to 32,000 cP.

Like viscosity, pH was essentially constant over the 12 week stability period. At 5°C storage the acne gel pH remained essentially constant, while at 25°C and 45°C a small increase of about 0.2 unit was recorded (Figure 2).

### Example 9: Crème brûlée skin renewal treatment formulation

A renewal treatment for dry, slack, rough, and/or wrinkled skin was prepared containing the ingredients and amounts shown in Table 4. This formula was made by preparing Phase A with moderate mixing, followed by separate preparation of Phase B, adjusting the pH with ammonium hydroxide to a pH of 3.8-4.2. Then, Phase B was mixed in to Phase A, and the resulting blend was heated to 75°C. In a different beaker, the ingredients of Phase C were combined and heated to 75°C. Then, Phase A-B and Phase C were combined and mixed for 5 minutes. The combination then was homogenized to 65°C-70°C, followed by mixing. After this step, Phase D was prepared and added to the combination of Phases A-B-C. When the final product cooled to 40°C, mixing was stopped, and allowed to thicken overnight.

The crème brûlée skin renewal treatment formula had a final appearance of a smooth, off-white cream / gel. The viscosity, measured by a Brookfield RVT viscometer using spindle T-C at 10 rpm, was 40,000 cP - 42,000 cP.

**Table 4: Crème brûlée skin renewal formulation of Example 9**

| **ingredient** | **addition level (% w/w)** |
|---|---|
| Phase A | |
| deionized water | 36.6 |
| lightly- to moderately-crosslinked PVP | 3.5 |
| propylene Glycol | 2.0 |
| disodium EDTA | 0.1 |
| *total* | 42.2 |
| | |
| Phase B | |
| deionized water | 20.0 |
| glycolic acid (70% active solution) | 11.4 |
| citric acid, anhydrous USP | 2.0 |
| ammonium hydroxide (28% active solution) | 2.8 |
| *total* | 36.2 |
| | |
| Phase C | |
| dicetyl phosphate, ceteth-10 phosphate | 3.5 |
| cetearyl alcohol | 2.5 |
| isodecyl neopentanoate | 2.5 |
| isocetyl stearate | 2.0 |
| decyl oleate | 2.25 |
| shea butter | 0.75 |
| dimethicone | 0.75 |
| *total* | 14.25 |
| | |
| Phase D | |
| disodium lauriminodipropionate tocopheryl phosphates | 0.75 |
| diazolidinyl urea and iodopropynyl butylcarbamate | 0.6 |
| Collaxyl | 2.0 |
| Orsirtine | 1.0 |
| Achromaxyl IS | 3.0 |
| *total* | 7.35 |
| ***grand total*** | **100.0** |

### Example 10: Reduced sting with tartaric acid solution

An independent, third-party clinical laboratory evaluated sting as a consumer perception of irritation for two formulations. The first formula was a 0.5% tartaric acid aqueous solution, and the second formula was an example of the invention, being identical to the first except it additionally contained 5% lightly- to moderately-crosslinked PVP. The facial discomfort assay test was conducted as a double-blind, crossover study. The formulas were applied to the faces of ten healthy, adult woman aged 21-67 previously tested and known to exhibit skin sensitivity to lactic acid. Prior to testing the abovedescribed two formulas, the volunteers' faces were washed with a standard, commercial beauty preparation, then gently patted dry. Approximately 1.0 mL of the two formulas was separately dispensed onto cotton swabs and liberally spread in smooth motions across the upper cheek area. Volunteers were instructed to record the discomfort/sting intensity of the two formulas after 2.5 and 5 minutes using the scale of Table 5. Additionally, the volunteers recorded all physical sensations. Relevant discomfort responses include: burning, stinging, tingling, itching, drying, smarting, prickly, and warm/hot. The evaluation method followed that described in Frosch, P.J. and Kligman, A.M., "A method for appraising the stinging capacity of topically applied substances," J. Soc Cos Chem, 28, p. 197-209 (1977), which hereby is incorporated in its entirety by reference.

In its written report, the independent, third-party laboratory concluded that formula 1 (without lightly- to moderately-crosslinked PVP) may be considered as stinging, while formula 2 (with lightly- to moderately-crosslinked PVP) may be considered as non-stinging. The mean numerical scale rating for the first formula was 0.68, and the mean numerical scale rating for the second formula (with lightly- to moderately-crosslinked PVP) was 0.18 (Table 6). Seven of the women did not sense any discomfort or irritation from the second formula (with lightly- to moderately-crosslinked PVP).

**Table 5: Discomfort/sting intensity scale used in Example 10**

| **numerical scale rating** | **volunteer perception** |
|---|---|
| 0 | none |
| 0.5 | barely perceptible |
| 1.0 | slightly perceptible |
| 1.5 | definitely perceptible |
| 2.0 | moderately perceptible |
| 2.5 | dramatically perceptible |
| 3.0 | severely perceptible |

**Table 6: Numerical scale rating results for the independent, third-part evaluation of Example 10.**

| **volunteer** | **formula 1: without lightly- to moderately-crosslinked PVP** | | | **formula 2: with lightly- to moderately-crosslinked PVP** | | |
|---|---|---|---|---|---|---|
| | **2.5 min** | **5.0 min** | **mean** | **2.5 min** | **5.0 min** | **mean** |
| 1 | 0 | 1.0 | 0.5 | 0 | 0 | 0 |
| 2 | 0,5 | 0.5 | 0.5 | 0 | 0 | 0 |
| 3 | 1.0 | 0 | 0.5 | 0.5 | 0 | 0.25 |
| 4 | 0 | 1.0 | 0.5 | 0 | 0 | 0 |
| 5 | 1.0 | 0 | 0.5 | 1.0 | 0.5 | 0.75 |
| 6 | 1.0 | 1.0 | 1.0 | 0 | 0 | 0 |
| 7 | 1.0 | 0.5 | 0.75 | 0 | 0 | 0 |
| 8 | 1.0 | 1.0 | 1.0 | 0 | 1.5 | 0.75 |
| 9 | 1.0 | 0 | 0.5 | 0 | 0 | 0 |
| 10 | 1.0 | 1.0 | 1.0 | 0 | 0 | 0 |
| **mean:** | | | **0.68** | | | **0.18** |
| **standard deviation:** | | | **0.24** | | | **0.32** |

### Example 11: Reduced sting with salicylic acid solution

Example 10 was repeated except salicylic acid replaced tartaric acid in both formula 1, the control (without lightly- to moderately-crosslinked PVP) and formula 2, the composition of the invention (with lightly- to moderately-crosslinked PVP). The concentration of salicylic acid in Example 11 was 0.5% (w/w) in both solutions.

In its written report, the independent, third-party laboratory concluded that formula 1 (without lightly- to moderately-crosslinked PVP) may be considered as stinging, while formula 2 (with lightly- to moderately-crosslinked PVP) may be considered as non-stinging. Less discomfort/sting and a more uniform volunteer perception was recorded by the volunteers for the formula of the example containing lightly- to moderately-crosslinked PVP (Table 7). Nine women did not sense any discomfort or irritation from the second formula (example of the invention),

**Table 7: Numerical scale rating results for the independent, third-part evaluation of Example 11.**

| **volunteer** | **formula 1: without lightly- to moderately-crosslinked PVP** | | | **formula 2: with lightly- to moderately-crosslinked PVP** | | |
|---|---|---|---|---|---|---|
| | **2.5 min** | **5.0 min** | **mean** | **2.5 min** | **5.0 min** | **mean** |
| 1 | 0 | 0 | 0 | 0 | 0 | 0 |
| 2 | 1.0 | 1.0 | 1.0 | 0 | 0 | 0 |
| 3 | 1.0 | 1.0 | 1.0 | 0 | 0 | 0 |
| 4 | 1.0 | 1.0 | 1.0 | 0 | 0 | 0 |
| 5 | 0 | 0 | 0 | 0.5 | 0.5 | 0.5 |
| 6 | 1.5 | 1.0 | 1.25 | 0 | 0 | 0 |
| 7 | 1.0 | 0 | 0.5 | 0 | 0 | 0 |
| 8 | 1.0 | 1.0 | 1.0 | 0 | 0 | 0 |
| 9 | 1.0 | 1.0 | 1.0 | 0 | 0 | 0 |
| 10 | 0 | 1.0 | 0.5 | 0.5 | 0 | 0 |
| **mean:** | | | **0.72** | | | **0.075** |
| **standard deviation:** | | | **0.45** | | | **0.16** |

### Example 12: Reduced sting with salicylic acid solution

Example 11 was repeated except a 2.0% salicylic acid solution replaced the 0.5% salicylic acid solution in both the control (without lightly- to moderately-crosslinked PVP) and the composition of the invention (with lightly- to moderately-crosslinked PVP).

Again, in its written report, the independent, third-party laboratory concluded that formula 1 (without lightly- to moderately-crosslinked PVP) may be considered as stinging, while formula 2 (with lightly- to moderately-crosslinked PVP) may be considered as non-stinging. Less discomfort/sting and a more uniform volunteer perception was recorded by the volunteers for the formula of the example containing lightly- to moderately-crosslinked PVP (Table 8).

**Table 8: Numerical scale rating results for the independent, third-part evaluation of Example 12.**

| volunteer | **formula 1: without lightly- to moderately-crosslinked PVP** | | | **formula 2: with lightly- to moderately-crosslinked PVP** | | |
|---|---|---|---|---|---|---|
| | **2.5 min** | **5.0 min** | **mean** | **2.5 min** | **5.0 min** | **mean** |
| 1 | 0 | 1.0 | 0.5 | 0 | 0 | 0 |
| 2 | 1.0 | 1.5 | 1.25 | 0 | 0 | 0 |
| 3 | 0 | 0 | 0 | 0.5 | 0 | 0.25 |
| 4 | 0 | 0 | 0 | 0 | 0.5 | 0.25 |
| 5 | 1.0 | 1.0 | 1.0 | 0 | 0.5 | 0.25 |
| 6 | 0.5 | 1.0 | 0.75 | | 0 | |
| 7 | 0 | 0 | 0 | 0 | 1.0 | 0.5 |
| 8 | 1.5 | 1.0 | 1.25 | 0 | 1.0 | 0.5 |
| 9 | 1.0 | 1.0 | 1.0 | 0 | 0 | 0 |
| 10 | 0 | 0 | 0 | 0.5 | 0.5 | 0.5 |
| **mean:** | | | **0.58** | | | **0.22** |
| **standard deviation:** | | | **0.54** | | | **0.22** |

## Claims

1. A composition having a pH of about 4 or lower and comprising:
(A) at least one personal care acid at 0.5% (% w/w) addition level or more, or one pharmaceutical acid at 0.5% (% w/w) addition level or more, wherein said personal care acid or pharmaceutical acid is an alpha and beta hydroxy acid or a beta hydroxy acid; and salts, esters or blends thereof; and
(B) lightly- to moderately-crosslinked PVP; wherein said PVP is present at from 1% to 6% by weight of the composition, has a gel volume of from 15 mL/g to 300 mL/g and a 5% solution thereof in a liquid carrier comprising water at 25°C has a Brookfield viscosity (measured in a Brookfield LVT or RVT viscometer) at 10 rpm of at least 2,000 cP;
wherein said composition has a Brookfield viscosity (measured in a Brookfield LVT or RVT viscometer) at 10 rpm and at 25°C using a T-C or T-E spindle of from 10,000 cP to 50,000 cP.

2. The composition of claim 1 wherein said addition level of either said personal care acid or said pharmaceutical acid is 1% or more.

3. The composition of claim 1 that has a pH of about 2 or lower.

4. The composition of claim 1 that is an anti-aging composition, a composition for skin blemishes, a smoothing composition, a moisturizing composition, a skin firming composition, a skin lightening composition, an age-spot composition, a shampoo, or a cream for use around the eyes or mouth.

5. The composition of claim 1 wherein:
(a) said beta hydroxy acid is selected from the group consisting of: beta hydroxybutanoic acid, tropic acid, trethocanic acid, salicylic acid, 5-(*n*-octanoyl) salicylic acid, and blends thereof; and
(b) said alpha and beta hydroxy acid is selected from the group of consisting of: citric acid, malic acid, tartaric acid, and blends thereof.

6. The composition of claim 1 having the form of: a solution, a cream, an ointment, a lotion, an oil-in-water emulsion, a water-in-oil emulsion, a shampoo, a spray, a gel, an aerosol, a suspension, a paste, a powder, a serum, or a mousse.

7. The composition of claim 1 that further comprises at least one additional ingredient selected from the group consisting of: active ingredients, emollients, liquid carriers, surfactants, emulsifiers, rheology modifiers, lubricants, diluents, humectants, anti-oxidants, preservatives, antibiotics, and blends thereof, optionally wherein said liquid carrier is selected from the group consisting of: water, alcohols, oils, esters, and blends thereof.

8. A composition as defined in claim 1 for use in a method of treatment of stinging perception on the skin, scalp, foot, or lip.

## Patentansprüche

1. Zusammensetzung mit einem pH von etwa 4 oder weniger, die Folgendes umfasst:
(A) zumindest eine Kosmetiksäure, die in einer Menge von 0,5 Gew.-% oder mehr zugesetzt ist, oder eine pharmazeutische Säure, die in einer Menge von 0,5 Gew.-% oder mehr zugesetzt ist, wobei die Kosmetiksäure oder pharmazeutische Säure eine α- und β-Hydroxysäure oder eine β-Hydroxysäure ist, sowie Salze, Ester oder Gemische davon; und
(B) schwach bis mäßig vernetztes PVP, wobei das PVP in einer Menge von 1 bis 6 Gew.-% der Zusammensetzung vorliegt, ein Gelvolumen von 15 ml/g bis 300 ml/g aufweist und eine 5-prozentige Lösung davon in einem flüssigen Träger, der Wasser umfasst, bei 25 °C eine Brookfield-Viskosität (gemessen in einem Brookfield-LVT- oder -RVT-Viskometer) von zumindest 2.000 cP bei 10 U/min aufweist;
wobei die Zusammensetzung unter Verwendung einer T-C- oder T-E-Spindel bei 10 U/min und 25 °C eine Brookfield-Viskosität (gemessen in einem Brookfield-LVT- oder -RVT-Viskometer) von 10.000 cP bis 50.000 cP aufweist.

2. Zusammensetzung nach Anspruch 1, wobei die zugesetzte Menge an Kosmetiksäure oder pharmazeutischer Säure 1 % oder mehr beträgt.

3. Zusammensetzung nach Anspruch 1, die einen pH von etwa 2 oder weniger aufweist.

4. Zusammensetzung nach Anspruch 1, die eine Anti-Age-Zusammensetzung, eine Zusammensetzung für Hautunreinheiten, eine glättende Zusammensetzung, eine feuchtigkeitsspendende Zusammensetzung, eine hautstraffende Zusammensetzung, eine hautbleichende Zusammensetzung, eine Altersflecken-Zusammensetzung, ein Shampoo oder eine Creme zur Verwendung um Augen oder Mund ist.

5. Zusammensetzung nach Anspruch 1, wobei:
(a) die β-Hydroxysäure aus der aus Folgendem bestehenden Gruppe ausgewählt ist: β-Hydroxybuttersäure, Tropasäure, Trethocansäure, Salicylsäure, 5-(n-Octanoyl)salicylsäure und Gemischen davon; und
(b) die α- und β-Hydroxysäure aus der aus Citronensäure, Äpfelsäure, Weinsäure und Gemischen davon bestehenden Gruppe ausgewählt ist.

6. Zusammensetzung nach Anspruch 1 in Form einer Lösung, einer Creme, einer Salbe, einer Lotion, einer ÖI-in-Wasser-Emulsion, einer Wasser-in-ÖI-Emulsion, eines Shampoos, eines Sprays, eines Gels, eines Aerosols, einer Suspension, einer Pasta, eines Pulvers, eines Serums oder eines Mousses.

7. Zusammensetzung nach Anspruch 1, die des Weiteren zumindest einen weiteren Inhaltsstoff, ausgewählt aus der aus Wirkstoffen, Emollienzien, flüssigen Trägern, Tensiden, Emulgatoren, Rheologiemodifikatoren, Gleitmitteln, Verdünnungsmitteln, Netzmitteln, Antioxidationsmitteln, Konservierungsmitteln, Antibiotika und Gemischen davon bestehenden Gruppe umfasst, wobei der flüssige Träger gegebenenfalls aus der aus Wasser, Alkoholen, Ölen, Estern und Gemischen davon bestehenden Gruppe ausgewählt ist.

8. Zusammensetzung nach Anspruch 1 zur Verwendung in einem Verfahren zur Behandlung eines brennenden Gefühls auf Haut, Kopfhaut, Füßen oder Lippe.

## Revendications

1. Composition ayant un pH d'environ 4 ou moins et comprenant :
(A) au moins un acide de soin personnel à un taux d'addition supérieur ou égal à 0,5 % (% m/m) ou un acide pharmaceutique à un taux d'addition supérieur ou égal à 0,5 % (% m/m), ledit acide de soin personnel ou ledit acide pharmaceutique étant un alpha et bêta-hydroxyacide ou un bêta-hydroxyacide ; et des sels, des esters ou des mélanges de ceux-ci ; et
(B) un PVP légèrement à modérément réticulé ; ledit PVP étant présent à hauteur de 1 % à 6 % en poids de la composition, ayant un volume de gel de 15 ml/g à 300 ml/g et une solution à 5 % de celui-ci dans un excipient liquide comprenant de l'eau à 25 °C ayant une viscosité Brookfield (mesurée par un viscosimètre LVT ou RVT Brookfield) à 10 tr/min d'au moins 2 000 cP ; ladite composition ayant une viscosité Brookfield (mesurée par un viscosimètre LVT ou RVT Brookfield) à 10 tr/min et à 25 °C en utilisant un mobile T-C ou T-E de 10 000 cP à 50 000 cP.

2. Composition selon la revendication 1, dans laquelle ledit taux d'addition dudit acide de soin personnel ou dudit acide pharmaceutique est supérieur ou égal à 1 %.

3. Composition selon la revendication 1, qui a un pH d'environ 2 ou moins.

4. Composition selon la revendication 1, qui est une composition anti-âge, une composition pour les imperfections cutanées, une composition lissante, une composition hydratante, une composition raffermissante pour la peau, une composition éclaircissante pour la peau, une composition antitâche de vieillissement, un shampooing ou une crème pour une utilisation autour des yeux ou de la bouche.

5. Composition selon la revendication 1, dans laquelle :
(a) ledit bêta-hydroxyacide est choisi dans le groupe constitué par l'acide bêta-hydroxybutanoïque, l'acide tropique, l'acide tréthocanique, l'acide salicylique l'acide 5-(*n-*octanoyl)salicylique et des mélanges de ceux-ci ; et
(b) ledit alpha et bêta-hydroxyacide est choisi dans le groupe constitué par : l'acide citrique, l'acide malique, l'acide tartrique et des mélanges de ceux-ci.

6. Composition selon la revendication 1, qui est sous la forme de : une solution, une crème, une pommade, une lotion, une émulsion huile-dans-l'eau, une émulsion eau-dans-l'huile, un shampooing, un spray, un gel, un aérosol, une suspension, une pâte, une poudre, un sérum ou une mousse.

7. Composition selon la revendication 1, qui comprend en outre au moins un ingrédient supplémentaire choisi dans le groupe constitué par : les principes actifs, les émollients, les excipients liquides, les tensioactifs, les émulsifiants, les modificateurs de rhéologie, les lubrifiants, les diluants, les humectants, les antioxydants, les conservateurs, les antibiotiques, et des mélanges de ceux-ci, éventuellement ledit excipient liquide étant choisi dans le groupe constitué par : l'eau, les alcools, les huiles, les esters et des mélanges de ceux-ci.

8. Composition telle que définie selon la revendication 1, destinée à être utilisée dans une méthode de traitement d'une perception de picotement sur la peau, le cuir chevelu, les pieds ou les lèvres.
